# EUROPEAN PATENT APPLICATION

(11) **EP 1 300 120 A1**
(43) Date of publication of application: **09.04.2003**
(21) Application number: 01123996.9
(22) Date of filing: 08.10.2001
(51) Int. Cl.: A61F 2/06

(54) **Stent delivery system**

(71) Applicant: JOMED NV, 6235 NS Ulestraten (NL)
(72) Inventor: Rueeger, Irene, 8164 Bachs (CH); Schaffner, Silvio, 8267 Berlingen (CH); Ha, Suk-Woo, 8246 Langwiesen (CH)
(74) Representative: Schmitz, Hans-Werner, Dipl.-Ing.

(57) **Abstract**

The present invention provides a stent holder (1) for self-expandable stents (2). In order to prevent the stent from migrating on a holder body (3) of the stent holder (1), said holder body (3) comprises two fixing means (4, 5) for temporarily fastening opposite stent ends (6, 7) of stent (2).

## Description

The present invention concerns a stent holder for self-expandable stents according to the preamble part of claim 1.

Such a stent holder comprises a holder body upon which the stent is temporarily disposed in its contracted state. An outer sheath is shifted over the stent in order to hold the stent in its contracted position until the stent has been moved to the site of implantation. In order to implant the stent, the outer sheath is retracted so that the stent can self-expand into its supporting position, e. g. within the stenosis of a body vessel.

Especially when the stent on the stent holder is pushed forward to curved vessels, it tends to move or migrate on the holder body as it is not positively fixed thereon. So, high stresses may be created in the stent so that the stentcatheter unit may be stuck in the vessel. Additionally, advancement through tortuous anatomy may longitudinally compress the stent, thereby significantly increasing the force required to release the stent. Unwanted compression may also occur during crimping or mounting.

It is therefore an object of the present invention to provide a stent holder according to the preamble part of claim 1 that is able to prevent the stent from moving or migrating when it is moved through body vessels, especially curved body vessels.

The solution of this object is achieved by the features of claim 1.

As the stent holder according to the present invention enables a fixation of the stent on at least one fixation element of the holder body, the stent body between its ends is flexibly supported on the holder body, and thus can easily follow a movement of the holder body through curved body vessels without a tendency to move or migrate.

So, the stent holder of the present invention is especially advantageous for long stents.

The fixation of the stent on the fixation element or the fixing means can e. g. be achieved by frictional forces or geometric constraint.

The dependent claims contain advantageous embodiments of the present invention.

So, the fixing means can be formed by two enlargements of the holder body. The positioning of the fixing means can be adapted to the length of the respective stent to be mounted on the holder body of the stent holder.

Preferably, the enlargements can be integral parts of the holder body.

However, an alternative are enlargements that are formed by glue layers to be applied to the holder body. With this embodiment, the positioning of the fixing means can easily be adapted to different stent lengths.

Further objects, advantages and features of the present invention become apparent from the description of the drawings in which:
- Fig. 1: is a schematically simplified side view of an embodiment of the stent holder of the present invention,
- Fig. 2: is a schematically simplified side view of the stent holder body of the stent holder of Fig. 1,
- Fig. 3: is a view corresponding to Fig. 2 of a part of the stent holder body depicting a first step of creation of a fixing means, and
- Fig. 4: is a view corresponding to Fig. 3 of a part of the stent holder body showing one embodiment of a fixing means in the form of a glue spot.

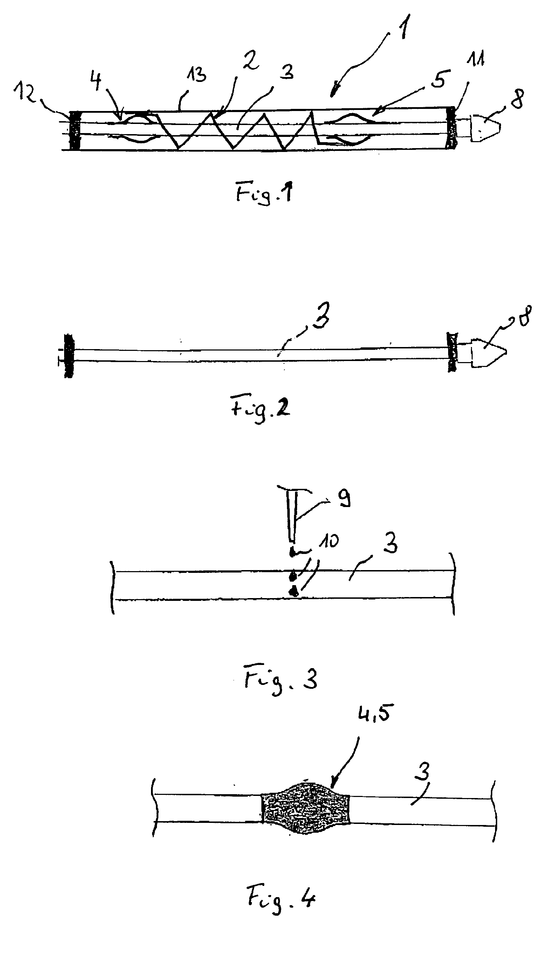

Fig. 1 shows an embodiment of the stent holder 1 of the present invention for a self-expandable stent 2. The stent holder 1 comprises an elongated holder body 3, preferably in the form of a circular cylindrical rod. The stent holder body 3 includes a body tip 8, two supporting means 11 and 12 and an outer sheath 13 that is pushed over the stent 2 in its contracted position in order to prevent the stent from expanding when mounted on the holder body 3.

Fig. 1 furthermore shows two fixation elements 4, 5 that are disposed on the holder body 3 spaced apart from each other. The distance between the fixing means 4, 5 depends on the stent length of the stent 2 such that the fixing means 4, 5 can temporarily hold the stent on holder body 3. An optimum spacing is a range between 1 cm to 10 cm.

Because of this fixation of stent 2 on holder body 3, stent 2 can be prevented from moving or migrating during a movement of the stent holder 1, particularly through curved body vessels. Additionally, longitudinal compression of the stent may be reduced during mounting or deployment, thereby reducing required mounting or delivery forces.

Figs. 2 and 3 show one exemplary technique for forming fixation elements 4, 5, seen in Fig. 4, from glue layers on the holder body 3. In Fig. 3, a tip 9 of a glueing device is shown that continuously drops a glue 10 onto the surface of holder body 3.

As per completing the form of the glue layer, as shown in Fig. 4, same could, for example, be heat-treated, UV cured, etc., in order to harden the glue spots.

As will be apparent to those of skill in the art, any number of fixation elements may be provided. Additionally, the spacing and/or size of the elements may be varied, as needed. Furthermore, additional techniques for forming fixation elements will be apparent, in addition to glue layers. For example, the elements could be formed from shrink tubing, could be formed integrally with holder body 3, or could otherwise be attached to the holder body. The appended claims are intended to cover all such changes or modifications that fall within the true spirit and scope of the present invention.

## Claims

1. A stent holder (1) for a stent (2), the stent holder (1) comprising an elongated holder body (3) having at least one fixation element (4, 5) for temporarily securing the stent (2) thereon.

2. The stent holder (1) of claim 1, wherein the holder body (3) has at least two fixation elements (4, 5) spaced apart from one another.

3. The stent holder (1) of claim 1, wherein the at least one fixation element comprises at least one enlargement of the holder body (3).

4. The stent holder (1) of claim 3, wherein the at least one enlargement is an integral part of the holder body (3).

5. The stent holder (1) of claim 3, wherein the at least one enlargement is formed from an enlargement chosen from the group consisting of glue layers, shrink tubing, an attachment to holder body (3), an integral portions of holder body (3), and combinations thereof.

6. The stent holder of claim 2, wherein the at least two fixation elements (4, 5) are spaced apart from one another by a distance in the range of 1 cm to 10 cm.
